(19) Europäisches Patentamt
European Patent Office
Office européen des brevets

(11) **EP 2 323 546 B1**

(12) **EUROPEAN PATENT SPECIFICATION**

(45) Date of publication and mention
of the grant of the patent:
**15.03.2017  Bulletin 2017/11**

(51) Int Cl.:
*A61B 5/0245* (2006.01)  *A61B 5/0205* (2006.01)
*A61B 5/024* (2006.01)  *A61B 5/08* (2006.01)
*A61B 5/05* (2006.01)

(21) Application number: **08813699.9**

(22) Date of filing: **15.09.2008**

(86) International application number:
**PCT/SG2008/000349**

(87) International publication number:
**WO 2010/030238 (18.03.2010 Gazette 2010/11)**

(54) **A METHOD FOR DETECTING HEARTBEAT AND/OR RESPIRATION**

VERFAHREN ZUM NACHWEIS VON HERZSCHLAG UND/ODER ATMUNG

PROCÉDÉ DE DÉTECTION DE LA PULSATION CARDIAQUE ET/OU DE LA RESPIRATION

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**25.05.2011  Bulletin 2011/21**

(73) Proprietor: **Nanyang Technological University
Singapore 639798 (SG)**

(72) Inventors:
• **SER, Wee**
**Singapore 459048 (SG)**
• **YU, Zhuliang**
**Guangdong (CN)**
• **YANG, Fan**
**Singapore 600279 (SG)**

(74) Representative: **Viering, Jentschura & Partner mbB**
**Patent- und Rechtsanwälte**
**Am Brauhaus 8**
**01099 Dresden (DE)**

(56) References cited:
WO-A2-03/005893  WO-A2-2004/013611
WO-A2-2007/124126  WO-A2-2007/143535
WO-A2-2008/057883  GB-A- 2 427 692
JP-A- 2001 260 698  JP-A- 2008 099 846
US-A1- 2006 170 584  US-A1- 2008 077 015

• **WEE SER ET AL: "Wearable system design with wheeze signal detection", MEDICAL DEVICES AND BIOSENSORS, 2008. ISSS-MDBS 2008. 5TH INTERNATIONAL SUMMER SCHOOL AND SYMPOSIUM ON, IEEE, PISCATAWAY, NJ, USA, 1 June 2008 (2008-06-01), pages 260-263, XP031341049, ISBN: 978-1-4244-2252-4**

Description

**FIELD OF THE INVENTION**

Embodiments of the invention relate to the field of electronic sensing, such as wireless sensing, for example. By way of example, embodiments of the invention relate to a method for detecting heartbeat and/or respiration, as well as a corresponding device.

**BACKGROUND OF THE INVENTION**

Electronic sensing has been used in the biomedical field for a long time, to perform functions such as monitoring the heartbeat rate and monitoring the respiratory rate, for example. Electronic sensing has also been used in electronic lie-detector systems, terrorist scanning, athlete health monitoring by monitoring a subject's pulse rate, for example.

US 2006/170584 A1 (C. E. Romero et. al.) discloses an obstacle penetrating dynamic radar imaging system for the detection, tracking, and imaging of an individual, animal, or object comprising a multiplicity of low power ultra wideband radar units that produce a set of return radar signals from the individual, animal, or object, and a processing system for said set of return radar signals for detection, tracking, and imaging of the individual, animal, or object. The system provides a radar video system for detecting and tracking an individual, animal, or object by producing a set of return radar signals from the individual, animal, or object with a multiplicity of low power ultra wideband radar units, and processing said set of return radar signals for detecting and tracking of the individual, animal, or object.

In most of the above mentioned examples, electronic sensing is typically performed via "wired" means. In this context, wires or cables are connected from a sensor attached to the subject's body to a processing system, which will process and interpret the electrical signals from the sensor in order to display a reading on the monitored characteristic (such as heartbeat rate, respiratory rate and pulse rate, for example). However, for some applications, "wired" means are difficult to implement or inconvenient for practical use.

Recently, electronic sensing via wireless means has been considered for use in estimating the heartbeat and/or respiratory rate, for example. In this context, one difficult problem encountered in estimating the heartbeat and/or respiratory rate using wireless means is that the reflected radio wave signal due to the heartbeat and the respiratory movement of chest is typically very weak. Although it is possible to increase the amplitude of the reflected signal by increasing the transmitted signal power, this has adverse effects on the health of the subject. Therefore, it is not a practical solution to increase the transmitted signal power.

Typically, electronic sensing in the biomedical field utilize radio wave signals with very low power.

To increase the probability of detecting the reflected radio signal within a same time interval, a high frequency radio signal can be used. This is because the frequency of the reflected signal is related to the frequency of the transmitted signal, where the doppler frequency of the reflected signal can be increased by increasing the frequency of the transmitted signal. However, a high frequency signal experiences strong attenuation while propagating in the human body. As a result, the performance of conventional methods on the detection and the subsequent analysis of this reflected radio wave signal is poor in such applications.

Additionally, it is difficult to get an accurate estimate of the heartbeat and/or respiratory rate, especially when there are movement perturbations between the sensor and the subject. These perturbations introduce additional random interference in the received signal. In some scenarios, these perturbations will even result in false detections of the heartbeat and/or respiratory rate.

It is also difficult to extract the heartbeat and/or respiratory rate of a specified target from crowd. The reflected signal contains the heartbeat/respiratory signal from almost all the subjects in the crowd. It is important to extract the reflected signal from a specific subject. This is especially difficult for conventional methods.

The above discussed problem may be addressed using embodiments of the present invention.

**SUMMARY OF THE INVENTION**

In an aspect of the invention, a method for detecting a bio-signal, the bio-signal being one of heartbeat or respiration, is provided. The method provided may include beamforming a transmit wave signal; transmitting the beamformed transmit wave signal to be reflected by a human body; beamforming a wave signal received by a plurality of antennas, wherein the received wave signal may include the reflected transmit wave signal that is modulated based on a perturbation and at least one of a movement of a heart wall or a chest wall of the human body; and analyzing the received wave signal with an analysis unit using a model for the bio-signal, thereby providing a result signal including an estimate of a rate of the bio-signal; wherein the analysis unit may be a Bayesian filter-like rate estimation module; and analyzing the received wave signal may include carrying out an estimation of the bio-signal with the Bayesian filter-like rate estimation module based on a known variance of the perturbation; and wherein the Bayesian filter-like rate estimation module may include an observation noise module, a signal model module, a state noise module, an adder module and a delay module.

In an aspect of the invention, a device for detecting a bio-signal, the bio-signal being one of heartbeat or respiration, may be provided. The device may include a transmitter unit including a plurality of antennas, the plurality of antennas configured to transmit a transmit wave signal to be reflected by a human body; a receiver unit including a plurality of antennas, the plurality of antennas configured to receive a wave signal, wherein the received wave signal may include the reflected transmit wave signal that is modulated based on a perturbation and at least one of a movement of a heart wall or a chest wall of the human body; a first beamforming unit configured to beamform the received reflected transmit wave signal; a second beamforming unit configured to beamform the transmit wave signal; and an analysis unit configured to analyze the received wave signal, thereby providing a result signal including an estimate of rate of the bio-signal; wherein the analysis unit may be a Bayesian filter-like rate estimation module including an observation noise module, a signal model module, a state noise module, an adder module and a delay module, wherein the analysis unit may be configured to carry out an estimation of the bio-signal with the Bayesian filter-like rate estimation module, based on a known variance of the perturbation.

In an example, a method for detecting heartbeat and/or respiration is provided. The method provided includes receiving a wave signal, and analyzing the received wave signal using a heartbeat and/or respiratory model, thereby providing a result signal indicating whether the received wave signal represents heartbeat and/or respiration.

In an example, a device for detecting heartbeat and/or respiration is provided. The device comprises a receiver unit configured to receive a wave signal, and an analysis unit configured to analyze the received wave signal using a heartbeat and/or respiratory model, thereby providing a result signal indicating whether the received wave signal represents heartbeat and/or respiration.

## BRIEF DESCRIPTION OF THE DRAWINGS

In the drawings, like reference characters generally refer to the same parts throughout the different views. The drawings are not necessarily to scale, emphasis instead generally being placed upon illustrating the principles of the invention. In the following description, various embodiments of the invention are described with reference to the following drawings, in which:

Figure 1A shows a flow chart of one method, according to one embodiment of the invention, for detecting heartbeat and/or respiration.

Figure 1B shows a system on which a method of detecting heartbeat and/or respiration may be implemented according to one embodiment of the invention.

Figure 2 shows a block diagram of a wireless heartbeat and respiratory rate monitoring (WHRM) device according to one embodiment of the invention.

Figure 3 shows a block diagram of the signal transmit/receive module (STRM) according to one embodiment of the invention.

Figure 4 shows a block diagram of a third configuration of the signal transmit/receive module (STRM) according to one embodiment of the invention.

Figure 5 shows a block diagram of the radio frequency (RF) module (RM) according to one embodiment of the invention.

Figure 6 shows a block diagram of the transmitter beamformer (TB) module according to one embodiment of the invention.

Figure 7 shows a block diagram of the receiver beamformer (RB) module according to one embodiment of the invention.

Figure 8 shows a block diagram illustrating the pre-processing block of the heartbeat and/or respiration detection and estimation module (HRDEM) according to one embodiment of the invention.

Figure 9 shows a block diagram of an implementation of the first method of estimating heartbeat and/or respiratory rate, according to one embodiment of the present disclosure.

Figure 10    shows a block diagram of an implementation of the second method of estimating heartbeat and/or respiratory rate, according to one embodiment of the present disclosure.

Figure 11    shows a block diagram of an implementation of the third method of estimating heartbeat and/or respiratory rate, according to one embodiment of the invention.

Figure 12    shows a block diagram of an implementation of the Bayesian filter-like rate estimation module, according to one embodiment of the invention.

Figure 13    shows a system built according to one embodiment of the invention.

Figure 14    shows a block diagram of various functional blocks employed by a signal processing software operating from a computer.

## DETAILED DESCRIPTION OF THE INVENTION

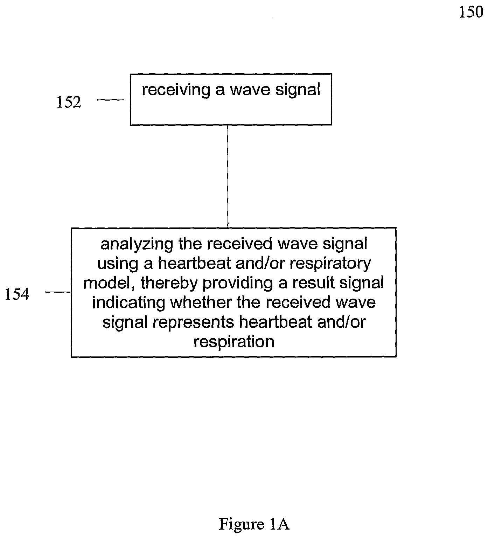

According to an embodiment of the invention, methods for detecting heartbeat and/or respiration are provided. **Figure 1A** shows a flow-chart 150 of one method for detecting heartbeat and/or respiration. The method includes receiving a wave signal at 152, and at 154 analyzing the received wave signal using a heartbeat and/or respiratory model, thereby providing a result signal indicating whether the received wave signal represents heartbeat and/or respiration. The method may include (not shown), before receiving the wave signal at 152, transmitting the wave signal. The method may also include providing an estimate of respiratory and/or heartbeat rate.

According to an embodiment of the invention, a device for detecting heartbeat and/or respiration is provided. The device provided includes a receiver unit configured to receive a wave signal, and an analysis unit configured to analyze the received wave signal using a heartbeat and/or respiratory model, thereby providing a result signal indicating whether the received wave signal represents heartbeat and/or respiration. The device may include a transmitter unit configured to transmit the wave signal for the receiver unit. The device may also provide an estimate of respiratory and/or heartbeat rate.

Embodiments of the invention emerge from the dependent claims.

Illustratively, the detection of the heartbeat and/or respiratory rate via wireless means may be carried out as follows. First, a wave signal may be transmitted to a subject. Next, the wave signal, which has been reflected from the subject, may be received. Finally, some signal processing techniques may be applied to the received wave signal, in order to obtain an estimate of the heartbeat and/or respiratory rate, for example.

In view of the above, in one embodiment, the method provided may further include providing an estimate of respiratory and/or heartbeat rate.

In one embodiment, analyzing the received wave signal using a heartbeat and/or respiratory model includes carrying out a spectral transformation on a signal dependent from the received wave signal, and components of the transformed signal, the frequency of which is below a predefined frequency threshold, are used for providing the result signal. In another embodiment, the predefined frequency threshold is in a range from about 0.5 Hz to about 3 Hz. In yet another embodiment, the predefined frequency threshold is in a range from about 0.2 Hz to about 1 Hz. In one embodiment, the spectral transformation is a Fourier Transformation.

In one embodiment, analyzing the received wave signal using a heartbeat and/or respiratory model includes carrying out a regression analysis (e.g., an auto-regression analysis) on a signal dependent from the received wave signal, thereby generating regression parameters and carrying out a spectral transformation on the signal dependent from the received wave signal using the regression parameters, wherein components of the transformed signal, the frequency of which is in a predefined frequency range, are used for providing the result signal.

In one embodiment, the heartbeat and/or respiratory model comprises a Bayesian-filter like heartbeat and/or respiratory model. In another embodiment, the Bayesian-filter like heartbeat and/or respiratory model comprises an estimation method based on a Kalman filter, e.g., an extended Kalman filter.

In one embodiment, the Bayesian-filter like heartbeat and/or respiratory model comprises an estimation method based on an unscented Kalman filter. In another embodiment, the Bayesian-filter like heartbeat and/or respiratory model comprises an estimation method based on a Particle filter.

In one embodiment, analyzing the received wave signal using a heartbeat and/or respiratory model includes carrying out a transformation on a signal dependent from the received wave signal, determining an observation signal comprising the transformed signal and carrying out a statistical analysis on the observation signal, wherein the result signal is provided based on the result of the statistical analysis.

In one embodiment, the received wave signal is an electromagnetic wave signal. In another embodiment, the received wave signal is a continuous wave electromagnetic wave signal.

In one embodiment, the electromagnetic wave signal has a frequency in a range of a radio wave signal transmitted from a transmitter device. In this embodiment, the transmitter device may be, but is not limited to, a wave signal transmitter device, for example.

In one embodiment, the method provided further includes beamforming the received wave signal. In another embodiment, the method provided further includes transmitting a transmit wave signal to be reflected, wherein the received wave signal comprises the reflected wave signal.

In one embodiment, the received wave signal is the transmitted wave signal modulated by reflection. In another embodiment, the received wave signal is the transmitted wave signal reflected by a living being and modulated by the reflection.

In one embodiment, the transmitted wave signal is modulated based on the motion of the heart wall and the chest of the living being. In another embodiment, the received wave signal is the transmitted wave signal phase modulated by the reflection.

In one embodiment, the method provided further includes beamforming the transmitted wave signal.

In one embodiment, analyzing the received wave signal includes demodulating the received wave signal.

In one embodiment, the device provided further includes at least one antenna and a first beamforming unit configured to beamform the received wave signal.

In one embodiment, the device provided further includes a transmitter unit configured to transmit a wave signal to be reflected, wherein the received wave signal comprises the reflected wave signal.

In one embodiment, the device provided further includes a second beamforming unit configured to beamform the transmitted wave signal.

The embodiments which are described in the context of the method for detecting heartbeat and/or respiration are analogously valid for the respective devices, and vice versa.

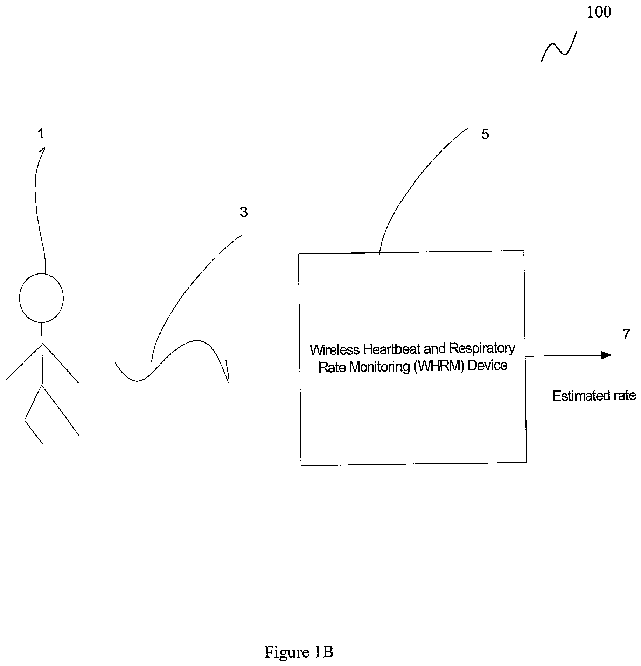

**Figure 1B** shows a system 100 on which a method of detecting heartbeat and/or respiration may be implemented according to one embodiment of the invention.

The system 100 includes a subject 1 and a wireless heartbeat and respiratory rate monitoring (WHRM) device 5. In this illustration, the method of detecting heartbeat and/or respiration may be implemented on the wireless heartbeat and respiratory rate monitoring (WHRM) device 5, for example.

The operation of the wireless heartbeat and respiratory rate monitoring (WHRM) device 5 may be described as follows.

The wireless heartbeat and respiratory rate monitoring (WHRM) device 5 first transmits a wave signal to the subject 1. In this context, the wave signal may be, but is not limited to, an electromagnetic wave signal, for example. In one embodiment, the wave signal is a continuous wave electromagnetic wave signal.

The wireless heartbeat and respiratory rate monitoring (WHRM) device 5 then receives another wave signal 3, where this received wave signal 3 may include the reflected transmitted wave signal from the subject 1, for example.

In one embodiment, the received wave signal 3 is the transmitted wave signal reflected by the subject 1 and modulated by the said reflection. In another embodiment, wherein the transmitted wave signal is modulated based on the motion of the heart wall and the chest of the subject 1. In this context, the received wave signal 3 may include information on the heartbeat and/or respiratory rate, for example.

The wireless heartbeat and respiratory rate monitoring (WHRM) device 5 then applies signal processing techniques to process the received wave signal 3, in order to obtain an estimate of the heartbeat and/or respiratory rate of the subject 1. The signal processing techniques applied will be described in more detail later.

Data on the heartbeat and/or respiratory rate is provided in a result signal 7, the result signal 7 also indicating whether the received wave signal 3 represents heartbeat and/or respiration.

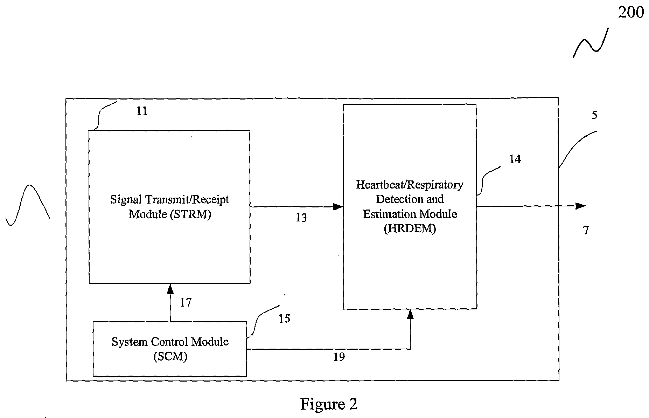

**Figure 2** shows a block diagram 200 of a wireless heartbeat and respiratory rate monitoring (WHRM) device 5 according to one embodiment of the invention.

As shown in **Figure 2,** the wireless heartbeat and respiratory rate monitoring (WHRM) device 5 includes a signal transmit/receipt module (STRM) 11, a heartbeat and/or respiration detection and estimation module (HRDEM) 14 and a system control module (SCM) 15.

The signal transmit/receive module (STRM) 11 performs the function of transmitting the wave signal to the subject 1. The signal transmit/receive module (STRM) 11 further performs the function of receiving the (reflected) wave signal, which may be reflected from the subject 1.

The signal transmit/receive module (STRM) 11 also provides a first signal 13 to the heartbeat and/or respiration detection and estimation module (HRDEM) 14. The first signal 13 may be, but is not limited to, a digitized signal which includes information on the heartbeat and/or respiratory rate of the subject 1, for example.

The heartbeat and/or respiration detection and estimation module (HRDEM) 14 performs the function of detecting and estimating the heartbeat and/or respiratory rate of the subject 1, based on the received first signal 13. The HRDEM 14 provides the results from detecting and estimating the heartbeat and/or respiratory rate of the subject 1 as data in the result signal 7.

The system control module (SCM) 15 provides a first control signal 17 to the signal transmit/receive module (STRM) 11 and a second control signal 19 to the heartbeat and/or respiration detection and estimation module (HRDEM) 14. The first control signal 17 and the second control signal 19 are used to respectively control the operations of the signal transmit/receive module (STRM) 11 and the heartbeat and/or respiration detection and estimation module (HRDEM) 14.

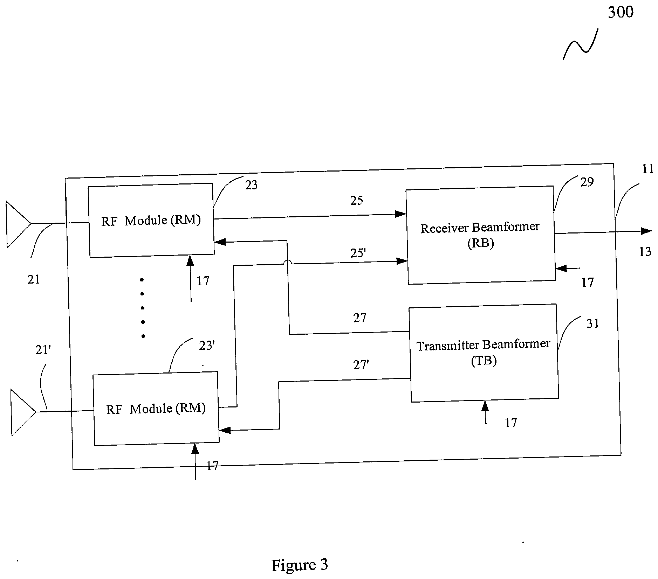

**Figure 3** shows a block diagram 300 of the signal transmit/receive module (STRM) 11 according to one embodiment of the invention.

As shown in **Figure 3,** the signal transmit/receive module (STRM) 11 includes an antenna 21, a radio frequency (RF) module (RM) 23, a receiver beamformer (RB) module 29 and a transmitter beamformer (TB) module 31.

In one embodiment, the signal transmit/receive module (STRM) 11 may include more than one antenna 21 and more than one radio frequency (RF) module (RM) 23. In this embodiment, the antenna is labeled as 21 and the additional antenna is labeled as 21'. Likewise, the radio frequency (RF) module (RM) is labeled as 23 and the additional radio frequency (RF) module (RM) is labeled as 23'.

In one embodiment, the antenna 21 may be identical to the additional antenna 21'. In another embodiment, the radio frequency (RF) module (RM) 23 may be identical to the additional radio frequency (RF) module (RM) 23'.

In this illustration, it can be seen that the wave signal 3 is in this embodiment an electromagnetic wave signal in the radio frequency (RF) range.

The antenna 21 may be used to transmit an RF wave signal (on the transmit path) and to receive the RF wave signal 3 (on the receive path).

The radio frequency (RF) module (RM) 23 may be used to process the RF wave signal to be transmitted on the transmit path. In this regard, a second signal 27 may be received from the transmitter beamformer (TB) module 31. The processed signal based on the second signal 27 may then be provided to the antenna 21, for transmission.

The radio frequency (RF) module (RM) 23 may be used to also process the received RF wave signal 3 on the receive path. In this regard, a processed wave signal 25 is provided by the radio frequency (RF) module (RM) 23 to the receiver beamformer (RB) module 29, for further processing.

The receiver beamformer (RB) module 29 may be used to form a beam to a desired direction (for example, the direction of the subject 1) so that the received RF wave signal 3 may be enhanced. The receiver beamformer (RB) module 29 receives the processed wave signal 25 from the radio frequency (RF) module (RM) 23, and provides the first signal 13 as its output signal.

Denoting the processed waveform signal 25 as $x_i(n)$, $i = 1, \cdots, L$, where $L$ is the number of sensors, the first signal 13, being the output signal is expressed as

$$y(n) = \sum_{i=1}^{L} \sum_{j=1}^{J} w_{ij} x_i(n-j)$$

the weights $w_{ij}$ is designed according to the spatial and temporal response of the STRM 11.

The transmitter beamformer (TB) module 31 may be used to generate the corresponding signals to feed the antenna 21, in order to form a transmission beam to a desired direction (for example, the direction of the subject 1).

Additionally, in the embodiment where the signal transmit/receive module (STRM) 11 may include more than one antenna 21 and more than one radio frequency (RF) module (RM) 23, there may be more than one second signal 27 and more than one processed wave signal 25. In this embodiment, the second signal is labeled as 27 and the additional second signal is labeled as 27'. Similarly, the processed wave signal is labeled as 25 and the additional processed wave signal is labeled as 25'.

In one embodiment, the second signal 27 may be identical to the additional second signal 27'. In another embodiment, the processed wave signal 25 may be identical to the additional processed wave signal 25'.

Further, it should be noted that the signal transmit/receive module (STRM) 11 may be arranged in different configurations.

In a first configuration, the signal transmit/receive module (STRM) 11 may be arranged as a multiple transmitter, multiple receiver system. In other words, the signal transmit/receive module (STRM) 11 may include a plurality of antennas 21 and a plurality of radio frequency (RF) modules (RM) 23, as shown in **Figure 3.**

In this configuration, with the use of the receiver beamformer (RB) module 29 and the transmitter beamformer (TB) module 31, the transmitted RF wave signal may be focused and directed towards the subject 1, and the received RF wave signal may be received by the plurality of antennas 21 with high directivity and a high antenna gain (i.e., with an enhanced signal to noise ratio (SNR)).

It can be seen that by controlling the receiver beamformer (RB) module 29 and the transmitter beamformer (TB) module 31 accordingly, this configuration has an advantage of being flexible in relation to signal transmission and signal reception.

However, this configuration has a disadvantage in that it has high implementation complexity as well as high implementation costs.

In a second configuration, the signal transmit/receive module (STRM) 11 may be arranged as a single transmitter, multiple receiver system. In this configuration, the signal transmit/receive module (STRM) 11 may include a plurality of antennas 21 and a plurality of radio frequency (RF) modules (RM) 23, as shown in **Figure 3.** However, in this configuration, only one radio frequency (RF) modules (RM) 23 may have a receive path as well as a transmit path, while the other radio frequency (RF) modules (RM) 23 may have only a receive path (i.e., no transmit path).

The second configuration has an advantage in that the plurality of antennas 21 may be used to enhance the received RF wave signal 3, i.e., the received RF wave signal may be received by the plurality of antennas 21 with high directivity and a high antenna gain (i.e., with an enhanced signal to noise ratio (SNR)). Further, thins configuration may be used to focus on the received RF wave signal 3 from the subject 1 who may be in the midst of a crowd, for example. Additionally, the implementation costs for this configuration is lower than that for the first configuration.

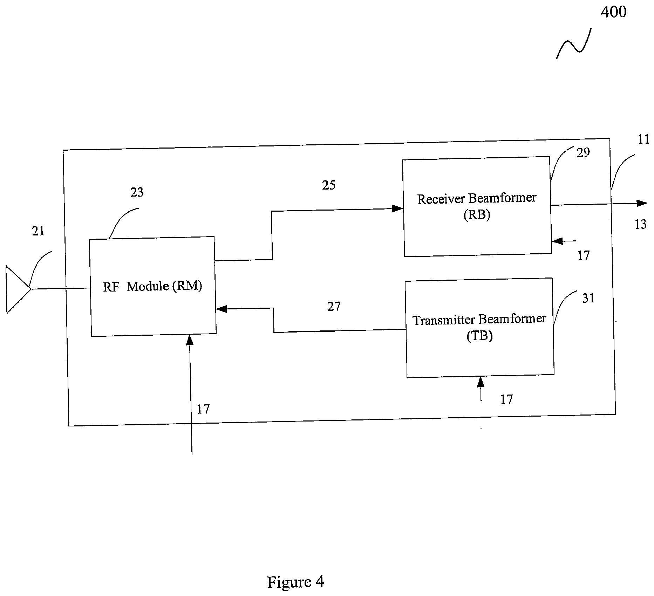

**Figure 4** shows a block diagram 400 of a third configuration of the signal transmit/receive module (STRM) 11 according to one embodiment of the invention.

In a third configuration, the signal transmit/receive module (STRM) 11 may be arranged as a single antenna system, which includes one antenna 21 and one radio frequency (RF) module (RM) 23, as shown in **Figure 4.**

In this configuration, the receiver beamformer (RB) module 29 may be a direct connection from the processed wave signal 25 to the first signal 13, for example. In other words, the first signal 13 may be the processed wave signal 25.

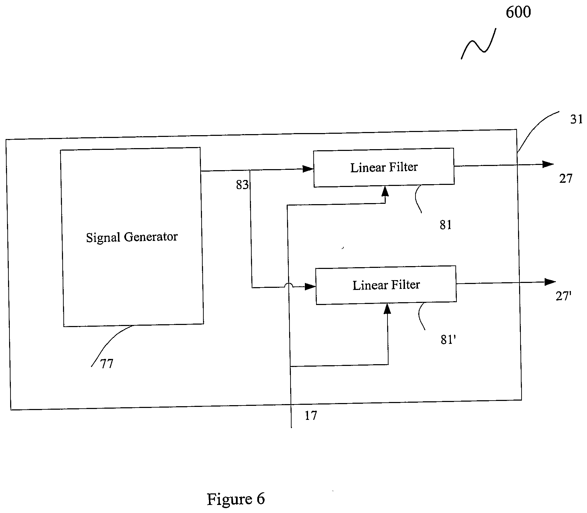

Further, in this configuration, the transmitter beamformer (TB) module 31 may be a single output system, for example. In this regard, the internal linear filters of transmitter beamformer (TB) module 31 (which will be discussed in more detail in relation to **Figure 6** later) may not be implemented.

The third configuration has an advantage in that it has a low implementation cost. However, the first configuration does not have the capability to enhance the received wave signal 3.

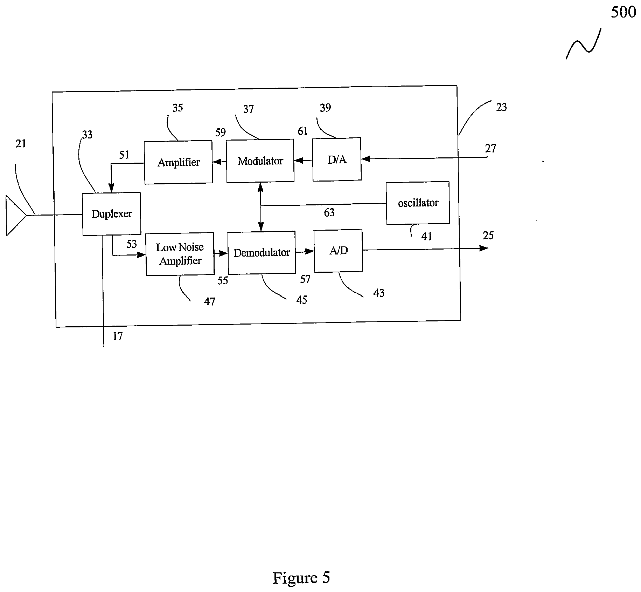

**Figure 5** shows a block diagram 500 of the radio frequency (RF) module (RM) 23 according to one embodiment of the invention.

As shown in **Figure 5,** the radio frequency (RF) module (RM) 23 includes a duplexer 33, a power amplifier 35, a modulator 37, a digital to analog converter (D/A) 39, an oscillator 41, an analog to digital converter (A/D) 43, a demodulator 45 and a low noise amplifier 47.

The duplexer 33 may be used to control the operation mode of the antenna 21. As mentioned earlier, the antenna 21 may be used to transmit the RF wave signal (on the transmit path) and to receive the RF wave signal 3 (on the receive path). In this context, for the transmit operation mode, the duplexer 33 may be used to switch the antenna 21 such that the antenna 21 is connected to the corresponding component on the transmit path, namely, the amplifier 35. On the other hand, for the receive operation mode, the duplexer 33 may be used to switch the antenna 21 such that the antenna 21 is connected to the corresponding component on the receive path, namely, the low noise amplifier 47.

The components along the transmit path may include the power amplifier 35, the modulator 37 and the digital to analog converter (D/A) 39. The signal flow along the transmit path may be described as follows.

The second signal 27, which is received by the radio frequency (RF) module (RM) 23 from the transmitter beamformer (TB) module 31, is a digital signal. The digital to analog converter (D/A) 39 converts the second signal 27 into an analog signal 61.

Next, the modulator 37 modulates the analog signal 61 onto a high frequency carrier signal 63 by, in order to obtain a resultant modulated signal 59. Following which, the power amplifier 35 amplifies the amplitude of the resultant modulated signal 59. Subsequently, the amplified signal 51 is fed to the antenna 21 via the duplexer 33.

As a side note, it can be seen from **Figure 5** that the high frequency carrier signal 63 is provided by the oscillator 41 to the modulator 37 as well as to the demodulator (on the receive path), i.e., the high frequency carrier signal 63 may be used on both the transmit and receive paths.

Further, it should be noted that the frequency of the high frequency carrier signal 63, $f_c$, may be selected according to the intended application, since the depth of penetration of the high frequency carrier signal varies depending on the frequency of the high frequency carrier signal. In one embodiment, the frequency of the high frequency carrier signal 63, $f_c$, may be in the range from about 100 MHz to 4 GHz, for example.

Turning now to the receive path, the components along the receive path may include the analog to digital converter (A/D) 43, the demodulator 45 and the low noise amplifier 47. The signal flow along the receive path may be described as follows.

A received signal 53 from the antenna 21 is provided to the low noise amplifier 47 via the duplexer 33. The low noise amplifier 47 amplifies the amplitude of the received signal 53.

Subsequently, the amplified received signal 55 is provided to the demodulator 45. The demodulator 45 processes the amplified received signal 55 using the reference high frequency signal 63, in order to retrieve a demodulated baseband signal 57. It should be noted that in general, the frequency components of the demodulated baseband signal 57 are usually of a significantly lower frequency compared to the frequency of the high frequency carrier signal, $f_c$.

Next, the analog to digital converter (A/D) 43 may be used to sample and convert the demodulated baseband signal 57 into the processed wave signal 25. In this context, the processed wave signal 25 may be a digital baseband signal, for example. The processed wave signal 25 may be used for further digital processing.

Further, the sampling process carried out by the analog to digital converter (A/D) 43 on the demodulated baseband signal 57 may be, but is not limited to, a single channel sampling process, or an I-Q channel sampling process, for example.

**Figure 6** shows a block diagram 600 of the transmitter beamformer (TB) module 31 according to one embodiment of the invention.

The transmitter beamformer (TB) module 31 includes a signal generator 77 and at least one linear filter 81.

In one embodiment, the transmitter beamformer (TB) module 31 may include more than one linear filter 81. In this embodiment, the linear filter is labeled as 81 and the additional linear filter is labeled as 81'. In another embodiment, the linear filter 81 may be identical to the additional linear filter 81'.

The signal generator 77 may be used to generate a transmit wave signal 83. The at least one linear filter 81 may receive and filter the transmit wave signal 83, and may provide the second signal 27.

Additionally, in the embodiment where the transmitter beamformer (TB) module 31 may include more than one linear filter 81, there may be more than one second signal 27. In this embodiment, the second signal is labeled as 27 and the additional second signal is labeled as 27'. In one embodiment, the second signal 27 may be identical to the additional second signal 27'.

As an illustrative example, in an embodiment where there are $N$ linear filters 81, the transmitter beamformer (TB) module 31 may generate $N$ second signals 27, in order to feed the antenna 21 to form a transmission beam to a desired direction, for example.

It should be noted that the response of the at least one linear filter 81 may be controlled using the first control signal 17. The response of the at least one linear filter 81 may be designed using conventional methods, for example.

As an illustrative example, the at least one linear filter 81 may be designed as a time-shift filter with different delay times. The response of such a linear filter may be expressed as

$$h(k) = \frac{\sin(\pi(k-D)T_s)}{\pi(k-D)T_s}, \quad k = 0, \cdots, K \qquad (1)$$

where $D$ is the time delay and $T_s$ is the sampling interval. Further, $K$ may be selected to be a value larger than $D$. It should be noted that the respective values to be used may be determined by experiment in order to achieve the desired performance.

Additionally, if a uniform linear array (ULA) is used, the delay time $D_i$ for the $i^{th}$ channel may be determined by

$$D_i = \frac{i \cdot d \cdot \cos\theta}{c} \qquad (2)$$

where $d$ is the inter-element distance of the array and $c$ is the speed of the radio wave signal.

The delay of each channel may then be determined. Next, the impulse response of each FIR filter $h(k)$ may be calculated, so as to steer the beam to the specific direction $\theta$.

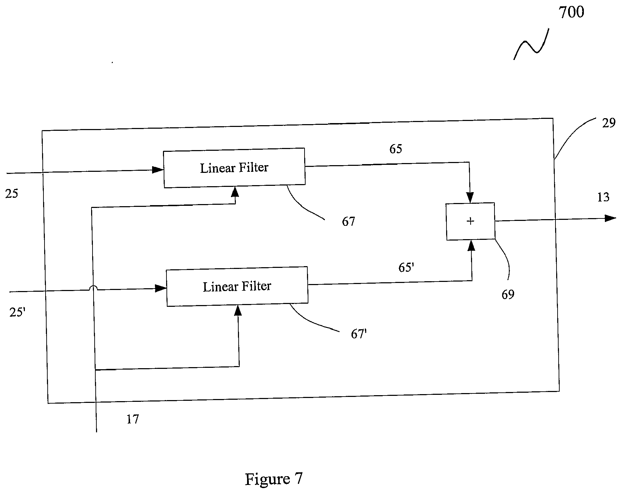

**Figure 7** shows a block diagram 700 of the receiver beamformer (RB) module 29 according to one embodiment of the invention.

The receiver beamformer (RB) module 29 includes at least one linear filter 67 and an adder unit 69.

In one embodiment, the receiver beamformer (RB) module 29 may include more than one linear filter 67. In this embodiment, the linear filter is labeled as 67 and the additional linear filter is labeled as 67'. In another embodiment, the linear filter 67 may be identical to the additional linear filter 67'.

Each of the at least one linear filter 67 may receive a processed wave signal 25 from a corresponding radio frequency (RF) module (RM) 23. Each of the at least one linear filter 67 may filter the processed wave signal 25 and may output a filtered wave signal 65.

The output signal (i.e. the first signal 13), $y(n)$, of the receiver beamformer (RB) module 29 is the weighted sum of the tap-delayed signal samples $x_i(n)$ of the processed wave signal 25, i.e.,

$$y(n) = \sum_{i=1}^{L} \sum_{j=1}^{J} w_{ij} x_i(n-j)$$

Additionally, in the embodiment where the receiver beamformer (RB) module 29 may include more than one linear filter 67, there may be more than one processed wave signal 25 and more than one filtered wave signal 65. In this embodiment, the processed wave signal is labeled as 25 and the additional processed wave signal is labelled as 25'. Likewise, the filtered wave signal is labeled as 65 and the additional filtered wave signal is labeled as 65'.

In one embodiment, the processed wave signal 25 may be identical to the additional processed wave signal 25'. In another embodiment, the filtered wave signal 65 may be identical to the additional filtered wave signal 65'.

The adder unit 69 may sum all the filtered wave signals 65 from the respective at least one linear filter 67, to form the first signal 13.

As an illustrative example, in an embodiment where there are *N* linear filters 67 (and correspondingly, *N* processed wave signals 25 and *N* filtered wave signals 65), the adder unit 69 may sum *N* filtered wave signals 65, to form the first signal 13.

It should be noted that the at least one linear filter 67 may be controlled using the first control signal 17. The response of the at least one linear filter 67 may be designed so as to maximize the signal to noise ratio (SNR) of the first signal 13, for example. The first control signal 17 may also be used to control the array beam pattern to specific direction, for example.

Further, the filter response of the at least one linear filter 67 may be fixed or adaptively adjusted.

Further, the first signal 13 provided to the heartbeat/respiratory detection and estimation module (HRDEM) 14 may include the Doppler frequency information of the heartbeat and/or respiratory rate.

Next, three different implementations of the heartbeat/respiratory detection and estimation module (HRDEM) 14 are discussed. In each implementation, different heartbeat and/or respiratory rate estimation methods may be used.

The first method is based on the Fast Fourier Transform (FFT) technique. This method has an advantage in that its implementation is quite simple and low cost. However, this method only has low frequency analysis resolution and may not be suitable for applications with perturbation between the equipment and the subject, e.g., a human.

The second method is based on the auto-regressive (AR) model for high resolution analysis. This method has higher frequency resolution. However, the frequency resolution provided by this method may still not be suitable for application with perturbation between the equipment and the human.

The third method is based on a state-space formulation of the signal model and the observed signal. In one embodiment, a Kalman filter like processing system may be used, in order to extract the heartbeat/respiratory rate with high resolution. This method has an advantage in that the high frequency resolution it provides may be able to deal with perturbation between the equipment and human. However, this method also has a disadvantage in that it has a high computation load compared to the other mentioned methods.

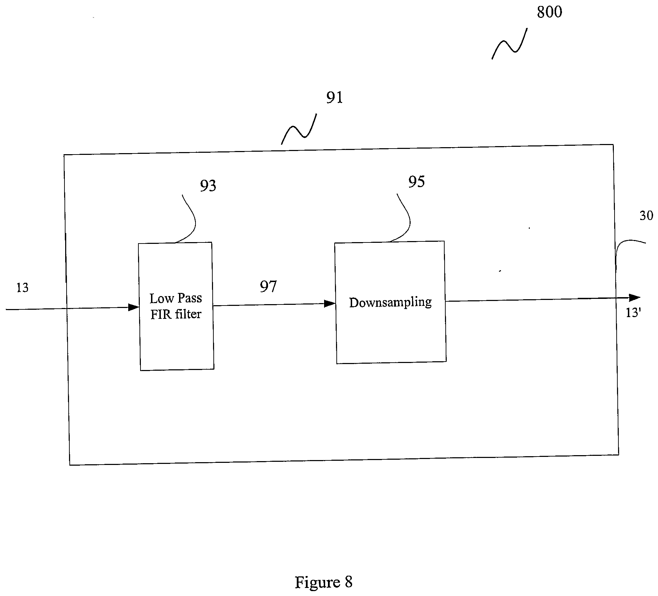

**Figure 8** shows a block diagram 800 illustrating the pre-processing block 91 of the heartbeat and/or respiration detection and estimation module (HRDEM) 14, according to one embodiment of the invention.

The pre-processing block 91 may be used to apply signal processing techniques on the first signal 13, in order to obtain a pre-processed signal 13'. In this context, the pre-processed signal 13' may be in a form which is more suitable for subsequent processing.

The pre-processing block 91 includes a low pass Finite Impulse Response (FIR) filter module 93 and a downsampling module 95.

The low pass Finite Impulse Response (FIR) filter module 93 may be used to remove the high frequency components in the first signal 13, in order to form a filtered first signal 97.

The downsampling module 95 may be used to reduce the sampling rate of the filtered first signal 97. The sampling rate of the filtered first signal 97 may be reduced by a factor of 2 or a factor of 5/4, for example.

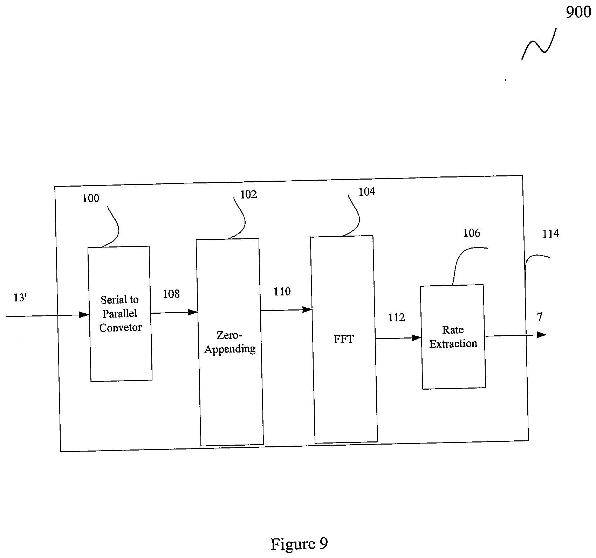

**Figure 9** shows a block diagram 900 of an implementation of the first method of estimating heartbeat and/or respiratory rate, according to one embodiment of the present disclosure.

As mentioned earlier, the first method of estimating heartbeat and/or respiratory rate may be based on the Fast Fourier Transform (FFT) technique.

The implementation of the first method of estimating heartbeat and/or respiratory rate includes a serial to parallel converter module 100, a zero appending module 102, a Fast Fourier Transform (FFT) module 104 and a rate extraction module 106.

The serial to parallel converter module 100 may convert the received pre-processed signal 13' into a signal block 108 with length N, for example.

The zero appending module 102 may then append the block signal 108 with N' zeros (at the end of the block signal) to form a new block signal 110. As a side note, the length of the new block signal 110 is *N+N'*.

Next, the Fast Fourier Transform (FFT) module 104 may apply a Fourier Transform operation on the new block signal 110, and provide an output signal 112 to the rate extraction unit 106. In mathematical expression, the Fourier Transform may be expressed as

$$X(k) = \sum_{n=0}^{N+N'-1} x(n)e^{-j\frac{2\pi}{N+N'}nk} \tag{3}$$

where $x(n)$ is the zero-appended signal vector. The index $k$ is the frequency index, i.e., the $k^{th}$ bin is equivalent to the frequency $kf_s/(N+N')$, where $f_s$ is the sampling rate.

As a side note, in practice, the calculation of Fourier Transform may be implemented in a more efficient form known as Fast Fourier Transform (FFT), for example.

The rate extraction module 106 may select the peak of the output signal 112 in low frequency band according to the sampling rate of the first signal 13' and the conventional frequency band of heartbeat and respiratory signal. The rate extraction module 106 provides an output being the result signal 7.

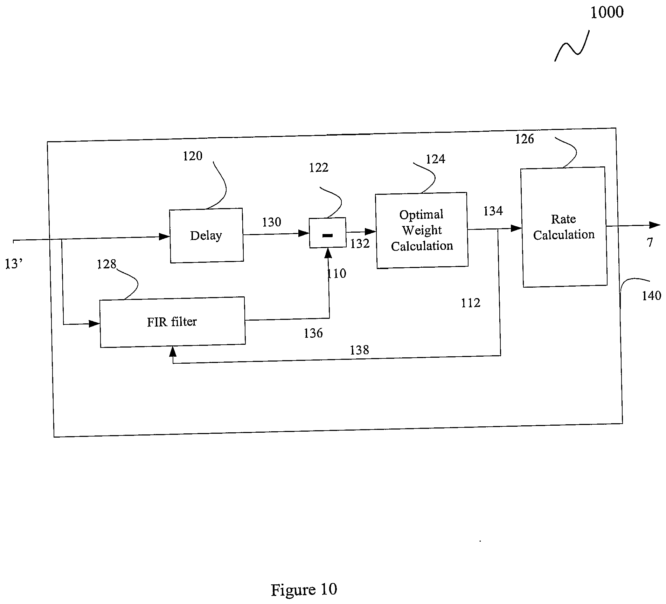

**Figure 10** shows a block diagram 1000 of an implementation of the second method of estimating heartbeat and/or respiratory rate, according to one embodiment of the present disclosure.

As mentioned earlier, the second method of estimating heartbeat and/or respiratory rate may be based on the auto-regressive (AR) model for high resolution analysis.

The implementation of the second method of estimating heartbeat and/or respiratory rate includes a delay module 120, a subtraction module 122, an optimal weight calculation module 124, a rate extraction module 126 and a Finite Impulse Response (FIR) filter module 128.

It is assumed that the received pre-processed signal 13' is a signal generated by an auto-regressive (AR) model. In this regard, the pre-processed signal 13', $x(n)$, may be expressed as

$$x(n) = \sum_{k=1}^{v} a_k x(n-k) + v(n) \tag{4}$$

where $\{a_k\}$ are the auto-regressive (AR) model parameters and $v(n)$ is the process noise.

The delay module 120 may receive the pre-processed signal 13', and may output a delayed first signal 130.

The Finite Impulse Response (FIR) filter module 128 may receive the pre-processed signal 13', and may output a third signal $y(n)$ 136. The third signal $y(n)$ 136 may be expressed as

$$y(n) = \sum_{k=1}^{v} a_k x(n-k) \tag{5}$$

The subtraction module 122 may subtract the third signal $y(n)$ 136 from the delayed first signal 130, to form an error signal, $e(n)$, 132, which may be expressed as

$$e(n) = x(n) - \sum_{k=1}^{p} a_k x(n-k) \tag{6}$$

The optimal weight calculation module 124 may calculate the filter coefficients of the Finite Impulse Response (FIR) filter module 128 (138). The algorithms used for calculating the filter coefficients of the Finite Impulse Response (FIR) filter module 128 (138), may be, but are not limited to, the linear prediction coefficient (LPC) algorithm, the least mean square (LMS) algorithm and the recursive least square (RLS) algorithm, for example.

The optimal weight calculation module 124 may also provide an optimal estimation of the auto-regressive (AR) model parameters $\{a_k\}$ 134. The algorithm used for providing the optimal estimation of the auto-regressive (AR) model parameters $\{a_k\}$ 134, may be, but are not limited to, the linear prediction coefficient (LPC) algorithm, the least mean square (LMS) algorithm and the recursive least square (RLS) algorithm, for example.

As an illustrative example, using the least mean square (LMS) algorithm, the optimal estimation of the auto-regressive (AR) model parameters $\{a_k\}$ 134 may be obtained as

$$a_k \leftarrow a_k + \mu e(n)x(n-k) \tag{7}$$

where $\mu$ is the step size.

The other algorithm used for providing the optimal estimation of the auto-regressive (AR) model parameters $\{a_k\}$ 134, such as the linear prediction coefficient (LPC) algorithm and the recursive least square (RLS) algorithm, for example, may also be applied in specific applications, such as in a reduced computational load scenario or in block processing mode, for example.

The rate calculation module 126 receives the calculated auto-regressive (AR) model parameters $\{a_k\}$ 134 and computes the spectrum of the first signal 13' as

$$P(\omega) = \frac{\sigma_n^2}{\displaystyle\sum_{k=1}^{p} a_k e^{j\omega T}} \tag{8}$$

where $\sigma_n^2$ is the variance of process noise and $T$ is the sampling rate.

The heartbeat and/or respiratory rate may then be extracted from the power spectrum $P(\omega)$ of the result signal 7. In this context, the spectrum peaks correspond to the heartbeat and/or respiratory rate.

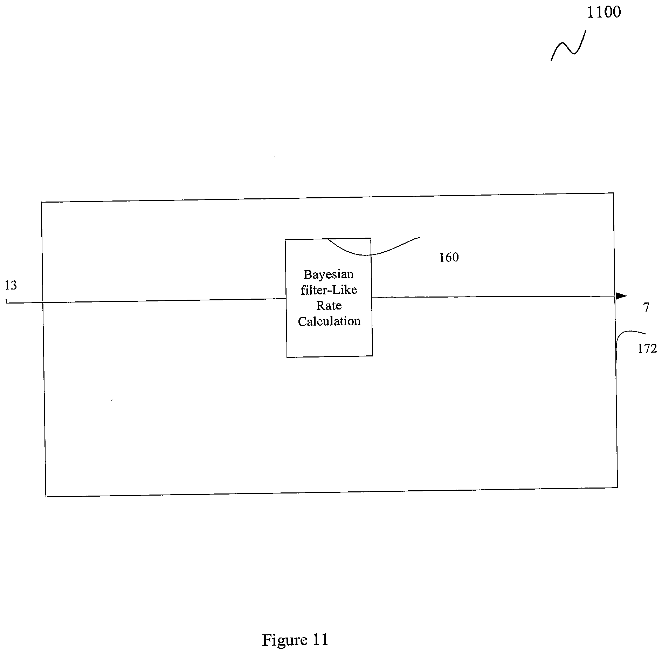

**Figure 11** shows a block diagram 1100 of an implementation of the third method of estimating heartbeat and/or respiratory rate, according to one embodiment of the invention.

As mentioned earlier, the third method of estimating heartbeat and/or respiratory rate may be based on a state-space formulation of the signal model and the observed signal.

The implementation of the third method of estimating heartbeat and/or respiratory rate includes a Bayesian filter-like rate estimation module 160. The Bayesian filter-like rate estimation module 160 accepts as input the first signal 13 and produces as output the result signal 7.

In this implementation, it is assumed that there are perturbations between the subject 1 and the antenna 21. Therefore, the resultant Doppler frequency information in the received signal wave 3 includes the frequency components not only caused by heartbeat and/or respiration, but also the perturbation between the subject 1 and the antenna 21.

The said perturbations are not known typically during the processing. However, the variance of the said perturbations may be estimated by experiment or may be given by prior knowledge. As such, this implementation may be able to estimate the heartbeat and/or respiratory rate with the unknown perturbations.

Next, the signal model 160 for Bayesian filter-like rate estimation is discussed in more detail.

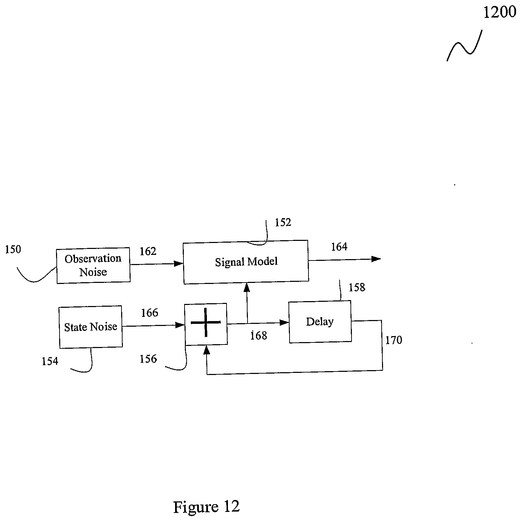

**Figure 12** shows a block diagram 1200 of an implementation of the Bayesian filter-like rate estimation module 160, according to one embodiment of the invention.

The Bayesian filter-like rate estimation module 160 includes an observation noise module 150, a signal model module 152, a state noise module 154, an adder module 156 and a delay module 158.

The observation noise module 150 may generate an observation noise signal $w(n)$ 162 and provide it to the signal model module 152.

The state noise module 154 may generate a perturbation signal $v(n)$ 166 and provide it to the adder module 156.

The adder module 156 may add the received perturbation signal $v(n)$ 166 to a delayed Doppler signal $f_h(n\text{-}1)$ 170, in order to obtain a Doppler signal $f_h(n)$ 168, i.e.,

$$f_h(n) = f_h(n-1) + v(n) \tag{9}$$

The Doppler signal $f_h(n)$ 168 may then be provided to the signal model 152 and the delay module 158. The Doppler signal $f_h(n)$ contains data on the heartbeat and/or respiratory rate.

The delay module 158 may generate the delayed Doppler signal $f_h(n\text{-}1)$ 170 from the received Doppler signal $f_h(n)$ 168, and provide it to the adder module 156.

The signal model module 152 may generate an observed signal $y(n)$ 164 based on the received observation noise signal $w(n)$ 162 and the received Doppler signal $f_h(n)$ 168. In more detail, the Doppler signal $f_h(n)$ 168 controls the auto-regressive (AR) model parameters $\{a_k\}$ of the observed signal $y(n)$ 164. In this regard, the observed signal $y(n)$ 164 may be expressed as

$$y(n) = g(f_h(n)) + w(n) \tag{10}$$

where $g(f_h(n))$ is a function of signal model 152. It will be appreciated that, from a signal processing view, the observed signal $y(n)$ 164 is equivalent to the pre-processed signal 13' of Figure 8.

As a side note, the function $g(f_h(n))$ is generally non-linear. Further, it should be noted that the Bayesian filter-like rate estimation method may be based on the observation signal $y(n)$ 164 and the signal model 152 $f_h(n)$.

In view that $y(n)$ is a nonlinear function of $f_h(n)$, methods of estimation such as the extended Kalman filter algorithm, the unscented Kalman filter algorithm or the Particle filter algorithm, for example, may be used to estimate $y(n)$. Further, since the function $f_h(n)$ is linear, and only the function $g(x)$ is non-linear, the Unscented Kalman filter algorithm may be a suitable choice to use as the method of estimation in practice.

The estimation of the frequency $f_h(n)$ may be described as follows.

**Initialization**

$$f_h(0) = 60 \quad \mathbf{P}(0) = 0.1 \tag{11}$$

It should be noted that any value may be used to the initial values. The initial values may be different from the ones shown in Equation (11).

Next, for the iterations $k \in 1, \cdots, \infty$, the following are calculated.

**Calculation of Sigma Points**

$$F(k-1) = \left[ \hat{f}_h(k-1) \quad \hat{f}_h(k-1) + \gamma\sqrt{\mathbf{P}(k-1)} \quad \hat{f}_h(k-1) - \gamma\sqrt{\mathbf{P}(k-1)} \right] \tag{12}$$

**Time Update**

$$F^*(k\,|\,k-1) = F(k-1)$$

$$\hat{f}_h^-(k) = \sum_{i=0}^{2L} W_i^{(m)} F_i^*(k\,|\,k-1)$$

$$\mathbf{P}^-(k) = \sum_{i=0}^{2L} W_i^{(c)} [F_i^*(k|k-1) - \hat{f}_h^-(k)][F_i^*(k|k-1) - \hat{f}_h^-(k)]^T + \sigma_v^2$$

$$F(k|k-1) = \left[ \hat{f}_h^-(k) \quad \hat{f}_h^-(k) + \gamma\sqrt{\mathbf{P}^-(k)} \quad \hat{f}_h^-(k) - \gamma\sqrt{\mathbf{P}^-(k)} \right]$$

$$Y_{(k|k-1)} = g(F(k|k-1))$$

$$\hat{y}_k^- = \sum_{i=0}^{2L} W_i^{(m)} Y_i(k\,|\,k-1)$$

$$\tag{13}$$

**Measurement Update**

$$\mathbf{P}_{\tilde{y}} = \sum_{i=0}^{2L} W_i^{(c)}[Y_i(k \mid k-1) - \hat{y}_k^-][Y_i(k \mid k-1) - \hat{y}_k^-]^T + \sigma_n^2$$

$$\mathbf{P}_{f\tilde{y}} = \sum_{i=0}^{2L} W_i^{(c)}[F_i^*(k|k-1) - \hat{f}^-(k)][Y_i(k|k-1) - \hat{y}_k^-]^T$$

$$K(k) = \mathbf{P}_{f\tilde{y}}\mathbf{P}_{\tilde{y}}^{-1} \qquad\qquad (14)$$

$$\hat{f}_h(k) = \hat{f}_h^-(k) + K(k)(y_k - \hat{y}_k^-)$$

$$\mathbf{P}(k) = \mathbf{P}^-(k) - K^2(k)\mathbf{P}_{\tilde{y}}$$

where $W_0^{(m)} = \frac{\lambda}{(L+\lambda)}$, $W_0^{(c)} = \frac{\lambda}{(L+\lambda)} + (1 - \alpha^2 + \beta)$ and $W_i^{(c)} = W_i^{(m)} = \frac{1}{2(\lambda+L)}, i = 1, \cdots, 2L$, and $\sigma_n^2, \sigma_v^2$ are respectively the variances of the observation noise and the process noise. The function $g(x) = \sin(2\pi xt)$ may be used in the iteration processing. The estimated heartbeat rate may be given by an estimate of $\hat{f}_h(k)$.

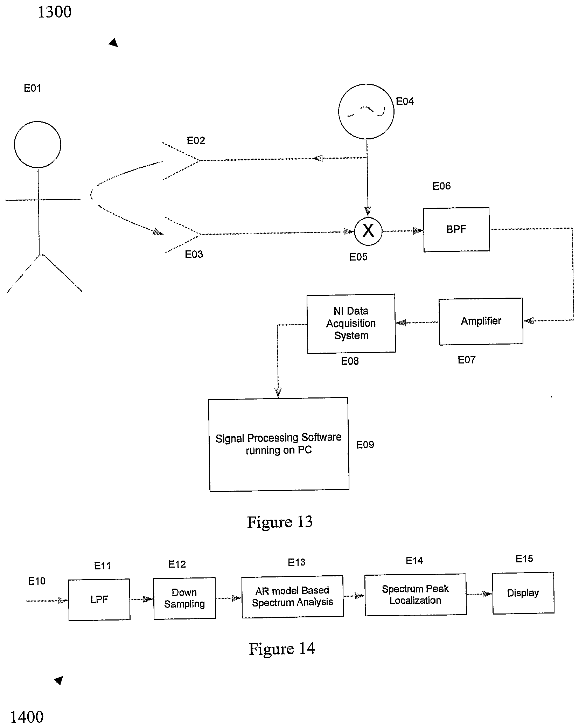

**Figure 13** shows a system 1300 built according to one embodiment of the invention.

In the system 1300, a single transceiver, is used, the transceiver using a transmitter unit E02 for RF signal transmitting, and a receiver unit E03 for receiving the wave signal reflected from a subject E01. A RF signal generator E04 outputs a sine wave operating at a frequency of, for example, 24GHz. The generated sine wave signal is transmitted from the transmitter unit E02. Part of the transmitted wave signal is reflected from the subject E01, where information on the heartbeat and/or respiration of the subject E01 is contained in the reflected signal.

Due to the movement of the heart wall, the chest wall and/or perturbation of the human body, the frequency of the reflected signal may be changed. The changed frequency is called the doppler frequency. This reflected signal is received by the receiver unit E03, and multiplied, in the multiplier E05, with a reference copy of the original transmitted signal transmitted from the transmitter unit E02.

The received wave signal (i.e. the reflected signal) undergoes filtering in the bandpass filter (BPF) E06, so that a signal containing data on the "doppler frequency" is output from the BPF E06. The output signal from the BPF E06 is amplified by the amplifier E07, which may in this embodiment, be a two-stage operational amplifiers with a gain of about 100. The amplified signal is fed to a data acquisition system E08 (such as one manufactured by [National Instrument (NI)], which is controlled by a signal processing software operating from a computer E09.

Figure 14 shows a block diagram 1400 of various functional blocks (E11 to E15) employed by the signal processing software operating from a computer E09 (see Figure 13).

The amplified signal from the amplifier E07 (see Figure 13) undergoes sampling, by the data acquisition system E08, at for example, a rate of 1 KHz and at 16bit ADC resolution.

The sampled digital signal E10 is first fed to a low-pass filter (LPF) E11, which is designed using a filter design toolbox in Matlab. After filtering, the signal is downsampled by a downsampling unit E12, by a factor of 100. In practical implementation, for downsampling factors of this magnitude, downsampling is preferably decomposed into several smaller factors, e.g., 100=5x5x4. For each filtering and downsampling procedure, only 5 or 4 times downsampling is implemented.

A downsampled signal with a sampling rate 10Hz is fed to an AR model based spectrum analysis unit E13. The order of the AR model based spectrum analysis unit E13 may be 4. With the estimated signal spectrum obtained by the AR model based spectrum analysis unit E13, spectrum peaks are located by the spectrum peak localization unit E14. The strongest peaks in the estimated signal spectrum are located, where the peak with high frequency provides data on the heartbeat of the subject E01 (see Figure 13), while the peak with low frequency provides data on the respiratory rate (see Figure 13). The peak locations are then transformed to the heartbeat and/or respiratory rate and shown in the display unit E15.

While the invention has been particularly shown and described with reference to specific embodiments, it should be understood by those skilled in the art that various changes in form and detail may be made therein without departing from the scope of the invention as defined by the appended claims. The scope of the invention is thus indicated by the appended claims and all changes which come within the meaning and range of equivalency of the claims are therefore intended to be embraced.

**Claims**

1.   A method for detecting a bio-signal, the bio-signal being one of heartbeat or respiration, the method comprising:

beamforming (E02) a transmit wave signal (27);
transmitting (E02) the beamformed transmit wave signal to be reflected by a human body (1);
beamforming (E03) a wave signal (13) received by a plurality of antennas (21), wherein the received wave signal (13) comprises the reflected transmit wave signal that is modulated based on a perturbation and at least one of a movement of a heart wall or a chest wall of the human body (1); and
analyzing (E13, E14) the received wave signal (13) with an analysis unit using a model for the bio-signal, thereby providing a result signal (7) comprising an estimate of a rate of the bio-signal;

**characterised in that** the analysis unit is a Bayesian filter-like rate estimation module (160); and
analyzing the received wave signal (13) comprises carrying out an estimation of the bio-signal with the Bayesian filter-like rate estimation module (160) based on a known variance of the perturbation; and
wherein the Bayesian filter-like rate estimation module (160) comprises an observation noise module (150), a signal model module (152), a state noise module (154), an adder module (156) and a delay module (158).

2. The method of claim 1,
wherein analyzing (E13, E14) the received wave signal (13) using a model for the bio-signal comprises carrying out a spectral transformation on a signal dependent from the received wave signal (13); and
wherein components of the transformed signal, the frequency value of which is below a predefined frequency threshold, are used for providing the result signal (7).

3. The method of claim 2,
wherein the spectral transformation is a Fourier Transformation.

4. The method of any one of claims 1 to 3,
wherein analyzing (E13, E14) the received wave signal (13) using the model for the bio-signal further comprises:

carrying out a spectral transformation on the signal dependent from the received wave signal (13) using the optimally-estimated regression parameters;
wherein components of the transformed signal, the frequency value of which is in a predefined frequency range, are used for providing the result signal (7).

5. The method of any one of claims 1 to 4, wherein the Bayesian filter-like rate estimation module (160) is configured to generate an observed signal based on any one of an extended Kalman filter, or on an unscented Kalman filter, or on a Particle filter algorithm.

6. The method of claim any one of claims 1 to 5,
wherein analyzing (E13, E14) the received wave signal (13)comprises

carrying out a transformation on a signal dependent from the received wave signal (13);
determining an observation signal comprising the transformed signal; and
carrying out a statistical analysis on the observation signal;

wherein the result signal (7) is provided based on the result of the statistical analysis.

7. The method of claim 6, wherein determining an observation signal comprises:

determining the observation signal from the transformed signal.

8. The method of any one of claims 1 to 7,
wherein the received wave signal (13) is an electromagnetic wave signal, and wherein the electromagnetic wave signal has a frequency in a range of a radio wave signal transmitted from a sensor device.

9. A device for detecting a bio-signal (5), the bio-signal being one of heartbeat or respiration, the device comprising:

a transmitter unit (11) comprising a plurality of antennas (21), the plurality of antennas configured to transmit a transmit wave signal (27) to be reflected by a human body (1);
a receiver unit (11) comprising a plurality of antennas (21), the plurality of antennas (21) configured to receive a wave signal (13), wherein the received wave signal (13) comprises the reflected transmit wave signal that is modulated based on a perturbation and at least one of a movement of a heart wall or a chest wall of the human body (1);
a first beamforming unit (29) configured to beamform the received reflected transmit wave signal;
a second beamforming unit (31) configured to beamform the transmit wave signal (27); and
an analysis unit (14) configured to analyze the received wave signal (13), thereby providing a result signal (7) comprising an estimate of rate of the bio-signal;

**characterised in that** the analysis unit is a Bayesian filter-like rate estimation module (160) comprising an observation noise module (150), a signal model module (152), a state noise module (154), an adder module (156) and a delay module (158),
wherein the analysis unit (14) is configured to carry out an estimation of the bio-signal with the Bayesian filter-like rate estimation module (160), based on a known variance of the perturbation.

10. The device (5) of claim 9,
wherein the Bayesian filter-like rate estimation module (160) is configured to generate an observed signal based on any one of an extended Kalman filter, an unscented Kalman filter, or a Particle filter algorithm.


**Patentansprüche**

1. Ein Verfahren zum Detektieren eines Biosignals, wobei das Biosignal eines von einem Herzschlag oder einer Atmung ist, wobei das Verfahren aufweist:

Strahlformen (E02) eines Übertragung-Wellensignals (27),
Übertragen (E02) des strahlgeformten Übertragung-Wellensignals, um von einem menschlichen Körper (1) reflektiert zu werden,
Strahlformen (E03) eines Wellensignals (13), das von einer Mehrzahl von Antennen (21) empfangen wird, wobei das empfangene Wellensignal (13) das reflektierte Übertragung-Wellensignal aufweist, welches moduliert wird basierend auf einer Perturbation und auf mindestens einer von einer Bewegung einer Herzwand oder einer Brustwand des menschlichen Körpers (1), und
Analysieren (E13, E14) des empfangenen Wellensignals (13) mit einer Analyseeinheit unter Verwendung eines Modells für das Biosignal, wodurch ein Ergebnissignal (7) bereitgestellt wird, welches eine Schätzung einer Rate des Biosignals aufweist,

**dadurch gekennzeichnet, dass**
die Analyseeinheit ein Bayes-Filter-ähnliches Rate-Schätzmodul (160) ist, und
das Analysieren des empfangenen Wellensignals (13) aufweist ein Ausführen einer Schätzung des Biosignals mit dem Bayes-Filter-ähnlichen Rate-Schätzmodul (160) basierend auf einer bekannten Varianz der Perturbation, und wobei das Bayes-Filter-ähnliche Rate-Schätzmodul (160) ein Überwachungsgeräuschmodul (150), ein Signalmodellmodul (152), ein Zustandsgeräuschmodul (154), ein Additionsmodul (156) und ein Verzögerungsmodul (158) aufweist.

2. Das Verfahren gemäß Anspruch 1,
wobei das Analysieren (E13, E14) des empfangenen Wellensignals (13) unter Verwendung eines Modells für das Biosignal aufweist:

ein Ausführen einer Spektraltransformation auf einem Signal abhängig von dem empfangenen Wellensignal (13), und

wobei Komponenten des transformierten Signals, dessen Frequenzwert unter einer vorbestimmten Frequenzschwelle liegt, zum Bereitstellen des Ergebnissignals (7) verwendet werden.

3. Das Verfahren gemäß Anspruch 2,
wobei die Spektraltransformation eine Fourier-Transformation ist.

4. Das Verfahren gemäß irgendeinem der Ansprüche 1 bis 3,
wobei das Analysieren (E13, E14) des empfangenen Wellensignals (13) unter Verwendung des Modells für das Biosignal ferner aufweist:

ein Ausführen einer Spektraltransformation auf dem Signal abhängig von dem empfangenen Wellensignal (13) unter Verwendung der optimal geschätzten Regressionsparameter,
wobei Komponenten des transformierten Signals, dessen Frequenzwert innerhalb eines vorbestimmten Frequenzbereichs liegt, zum Bereitstellen des Ergebnissignals (7) verwendet werden.

5. Das Verfahren gemäß irgendeinem der Ansprüche 1 bis 4, wobei das Bayes-Filter-ähnliche Rate-Schätzmodul (160) konfiguriert ist, um ein Überwacht-Signal zu erzeugen basierend auf irgendeinem von einem Extended-Kalman-Filter-Algorithmus, einem Unscented-Kalman-Filter-Algorithmus oder einem Partikel-Filter-Algorithmus.

6. Das Verfahren gemäß irgendeinem der Ansprüche 1 bis 5,
wobei das Analysieren (E13, E14) des empfangenen Wellensignals (13) aufweist:

ein Ausführen einer Transformation auf einem Signal abhängig von dem empfangenen Wellensignal (13),
Ermitteln eines Überwachungssignals, welches das transformierte Signal aufweist, und
ein Ausführen einer statistischen Analyse auf dem Überwachungssignal,

wobei das Ergebnissignal (7) bereitgestellt wird basierend auf dem Ergebnis der statistischen Analyse.

7. Das Verfahren gemäß Anspruch 6, wobei das Ermitteln eines Überwachungssignals aufweist:

ein Ermitteln des Überwachungssignals von dem transformierten Signal.

8. Das Verfahren gemäß irgendeinem der Ansprüche 1 bis 7,
wobei das empfangene Wellensignal (13) ein elektromagnetisches Wellensignal ist, und wobei das elektromagnetische Wellensignal eine Frequenz in einem Bereich eines Radiowellensignals hat, welches von einer Sensorvorrichtung übertragen wird.

9. Eine Vorrichtung zum Detektieren eines Biosignals (5), wobei das Biosignal eines von einem Herzschlag oder einer Atmung ist, wobei die Vorrichtung aufweist:

eine Übertragungseinheit (11), welche eine Mehrzahl von Antennen (21) aufweist, wobei die Mehrzahl von Antennen konfiguriert ist, um ein Übertragung-Wellensignal (27) zu übertragen, das von einem menschlichen Körper (1) reflektiert werden soll,
eine Empfangseinheit (11), welche eine Mehrzahl von Antennen (21) aufweist, wobei die Mehrzahl von Antennen (21) konfiguriert ist, um ein Wellensignal (13) zu empfangen, wobei das empfangene Wellensignal (13) das reflektierte Übertragung-Wellensignal aufweist, welches moduliert wird basierend auf einer Perturbation und auf mindestens einer von einer Bewegung einer Herzwand oder einer Brustwand des menschlichen Körpers (1),
eine erste Strahlform-Einheit (29), die konfiguriert ist, um das empfangene, reflektierte Übertragung-Wellensignal beamzuformen,
eine zweite Strahlform-Einheit (31), die konfiguriert ist, um das Übertragung-Wellensignal (27) strahlzuformen, und
eine Analyseeinheit (14), die konfiguriert ist, um das empfangene Wellensignal (13) zu analysieren, und dadurch ein Ergebnissignal (7) bereitzustellen, das eine geschätzte Rate des Biosignals aufweist,

**dadurch gekennzeichnet, dass**
die Analyseeinheit ein Bayes-Filter-ähnliches Rate-Schätzmodul (160) ist, welches ein Überwachungsgeräuschmodul (150), ein Signalmodellmodul (152), ein Zustandsgeräuschmodul (154), ein Additionsmodul (156) und ein Verzögerungsmodul (158) aufweist,
wobei die Analyseeinheit (14) konfiguriert ist, um eine Schätzung des Biosignals mit dem Bayes-Filter-ähnlichen Rate-Schätzmodul (160) auszuführen basierend auf einer bekannten Varianz der Perturbation.

10. Die Vorrichtung (5) gemäß Anspruch 9, wobei das Bayes-Filter-ähnliche Rate-Schätzmodul (160) konfiguriert ist, um ein Überwacht-Signal zu erzeugen basierend auf irgendeinem von einem Extended-Kalman-Filter-Algorithmus, einem Unscented-Kalman-Filter-Algorithmus oder einem Partikel-Filter-Algorithmus.

**Revendications**

1. Procédé de détection d'un biosignal, le biosignal étant l'un d'un battement cardiaque ou d'une respiration, le procédé comprenant :

   la formation de faisceau (E02) d'un signal d'onde de transmission (27) ;
   la transmission (E02) du signal d'onde de transmission dont le faisceau a été formé afin qu'il soit réfléchi par un corps humain (1) ;
   la formation de faisceau (E03) d'un signal d'onde (13) reçu par une pluralité d'antennes (21), le signal d'onde reçu (13) comprenant le signal d'onde de transmission réfléchi qui est modulé sur la base d'une perturbation et d'au moins l'un d'un mouvement d'une paroi cardiaque ou d'une paroi thoracique du corps humain (1) ; et
   l'analyse (E13, E14) du signal d'onde reçu (13) avec une unité d'analyse à l'aide d'un modèle pour le biosignal, afin d'obtenir un signal de résultat (7) comprenant une estimation d'une vitesse du biosignal ;

   **caractérisé en ce que**
   l'unité d'analyse est un module d'estimation de vitesse sous forme de filtre bayésien (160) ; et
   l'analyse du signal d'onde reçu (13) comprend la réalisation d'une estimation du biosignal avec le module d'estimation de vitesse sous forme de filtre bayésien (160) sur la base d'une variance connue de la perturbation ; et
   dans lequel le module d'estimation de vitesse sous forme de filtre bayésien (160) comprend un module de bruit d'observation (150), un module de modèle de signal (152), un module de bruit d'état (154), un module d'additionneur (156) et un module de retard (158).

2. Procédé selon la revendication 1,
   dans lequel l'analyse (E13, E14) du signal d'onde reçu (13) à l'aide d'un modèle pour le biosignal comprend la réalisation d'une transformation spectrale sur un signal en fonction du signal d'onde reçu (13) ; et
   dans lequel les composantes du signal transformé, dont la valeur de fréquence est inférieure à un seuil de fréquence prédéfini, sont utilisées pour fournir le signal de résultat (7).

3. Procédé selon la revendication 2,
   dans lequel la transformation spectrale est une transformée de Fourier.

4. Procédé selon l'une quelconque des revendications 1 à 3,
   dans lequel l'analyse (E13, E14) du signal d'onde reçu (13) à l'aide du modèle pour le biosignal comprend en outre :

   la réalisation d'une transformation spectrale sur le signal en fonction du signal d'onde reçu (13) à l'aide des paramètres de régression estimés de manière optimale ;
   dans lequel les composantes du signal transformé, dont la valeur de fréquence se trouve dans des limites de fréquence prédéfinies, sont utilisées pour fournir le signal de résultat (7).

5. Procédé selon l'une quelconque des revendications 1 à 4, dans lequel le module d'estimation de vitesse sous forme de filtre bayésien (160) est configuré pour générer un signal observé sur la base de n'importe lequel d'un filtre de Kalman étendu, ou d'un filtre de Kalman sans parfum, ou d'un algorithme de filtre à particules.

6. Procédé selon l'une quelconque des revendications 1 à 5, dans lequel l'analyse (E13, E14) du signal d'onde reçu (13) comprend
   la réalisation d'une transformation sur un signal en fonction du signal d'onde reçu (13) ;
   la détermination d'un signal d'observation comprenant le signal transformé ;
   et
   la réalisation d'une analyse statistique sur le signal d'observation ;
   dans lequel le signal de résultat (7) est fourni sur la base du résultat de l'analyse statistique.

7. Procédé selon la revendication 6, dans lequel la détermination d'un signal d'observation comprend :

   la détermination du signal d'observation à partir du signal transformé.

8. Procédé selon l'une quelconque des revendications 1 à 7,
   dans lequel le signal d'onde reçu (13) est un signal d'onde électromagnétique, et dans lequel le signal d'onde électromagnétique présente une fréquence de l'ordre de celle d'un signal d'onde radio transmis par un capteur.

9. Dispositif de détection d'un biosignal (5), le biosignal étant l'un d'un battement cardiaque ou d'une respiration, le dispositif comprenant :

une unité de transmission (11) comprenant une pluralité d'antennes (21), la pluralité d'antennes étant configurée pour transmettre un signal d'onde de transmission (27) qui doit être réfléchi par un corps humain (1) ;
une unité de réception (11) comprenant une pluralité d'antennes (21), la pluralité d'antennes (21) étant configurée pour recevoir un signal d'onde (13), le signal d'onde reçu (13) comprenant le signal d'onde de transmission réfléchi qui est modulé sur la base d'une perturbation et d'au moins l'un d'un mouvement d'une paroi cardiaque ou d'une paroi thoracique du corps humain (1) ;
une première unité de formation de faisceau (29) configurée pour former le faisceau du signal d'onde de transmission réfléchi reçu ;
une seconde unité de formation de faisceau (31) configurée pour former le faisceau du signal d'onde de transmission (27) ; et
une unité d'analyse (14) configurée pour analyser le signal d'onde reçu (13), afin d'obtenir un signal de résultat (7) comprenant une estimation de la vitesse du biosignal ;

**caractérisé en ce que**
l'unité d'analyse est un module d'estimation de vitesse sous forme de filtre bayésien (160) comprenant un module de bruit d'observation (150), un module de modèle de signal (152), un module de bruit d'état (154), un module d'additionneur (156) et un module de retard (158),
dans lequel l'unité d'analyse (14) est configurée pour réaliser une estimation du biosignal avec le module d'estimation de vitesse sous forme de filtre bayésien (160), sur la base d'une variance connue de la perturbation.

10. Dispositif (5) selon la revendication 9,
dans lequel le module d'estimation de vitesse sous forme de filtre bayésien (160) est configuré pour générer un signal observé sur la base d'un quelconque d'un filtre de Kalman étendu, d'un filtre de Kalman sans parfum, ou d'un algorithme de filtre à particules.

150

152 ——— | receiving a wave signal |

154 ——— analyzing the received wave signal
using a heartbeat and/or respiratory
model, thereby providing a result signal
indicating whether the received wave
signal represents heartbeat and/or
respiration

Figure 1A

100

1

3

5

Wireless Heartbeat and Respiratory
Rate Monitoring (WHRM) Device

7

Estimated rate

Figure 1B

200

| Signal Transmit/Receipt Module (STRM) 11 | 13 → | Heartbeat/Respiratory Detection and Estimation Module (HRDEM) 14 | 5 → 7 |

System Control Module (SCM) 15

17

19

Figure 2

300

RF Module (RM) 23

25

Receiver Beamformer
(RB) 29

11

21

17

25'

13

17

21'

23'

27

Transmitter Beamformer
(TB) 31

RF Module (RM)

27'

17

17

17

Figure 3

400

21 23 25

29 11

Receiver Beamformer
(RB)

13

17

RF Module (RM)

27

Transmitter Beamformer
(TB)

31

17

17

Figure 4

Figure 5

600

31

```
┌─────────────────────────────────────────────────────────┐
│                                                          │
│   ┌──────────────────┐                                   │
│   │                  │      ┌──────────────────┐         │──► 27
│   │                  │──────► │  Linear Filter   │──────►│
│   │                  │ 83     └──────────────────┘        │
│   │                  │              ▲         81          │
│   │ Signal Generator │              │                     │
│   │                  │      ┌──────────────────┐          │
│   │                  │──────► │  Linear Filter   │──────►│──► 27'
│   │                  │        └──────────────────┘         │
│   │                  │              ▲                      │
│   └──────────────────┘              │       81'            │
│          77                         │                      │
│                                     │                      │
└─────────────────────────────────────────────────────────┘
                                  17
```

Figure 6

700

29

Linear Filter

65

25

67

+ 13

69

Linear Filter

65'

25'

67'

17

Figure 7

800

91

93                                    95

                                                              30

13          Low Pass        97          Downsampling
            FIR filter                                              13'

Figure 8

900

Figure 9

1000

Figure 10

1100

Bayesian
filter-Like
Rate
Calculation

160

13

7

172

Figure 11

1200

150

| Observation Noise | 162 | Signal Model | 164 →

152

State Noise | 166 | + | 168 | Delay

158

154    156    170

Figure 12

1300

E01

E04

E02

E06

E03

E05

BPF

NI Data
Acquisition
System

Amplifier

E08

E07

Signal Processing Software
running on PC

E09

Figure 13

E10

E11
LPF

E12
Down
Sampling

E13
AR model Based
Spectrum Analysis

E14
Spectrum Peak
Localization

E15
Display

Figure 14

1400

**EP 2 323 546 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2006170584 A1, C. E. Romero